# EUROPEAN PATENT SPECIFICATION

(11) **EP 2 414 534 B1**
(45) Date of publication and mention of the grant of the patent: **06.11.2013**
(21) Application number: 10723267.0
(22) Date of filing: 30.03.2010
(51) Int. Cl.: C12Q 1/00, G01N 33/543

(54) **ELECTROCHEMICAL TEST DEVICE**
ELEKTROCHEMISCHE TESTVORRICHTUNG
DISPOSITIF DE TEST ÉLECTROCHIMIQUE

(30) Priority: 31.03.2009 GB 0905553
(43) Date of publication of application: 08.02.2012
(73) Proprietor: DIAMATRIX LIMITED, Cliddesden, Hampshire RG25 2JF (GB)
(72) Inventor: JACKSON, James, Richard, Hampshire RG25 2JF (GB); PALMER, Richard, F., Bucks SL2 3EH (GB)
(74) Representative: Forsythe, Dominic
(86) International application number: PCT/GB2010/000613
(87) International publication number: WO 2010/112833

(56) References cited:
- EP-A1- 1 712 919
- EP-A2- 1 443 327
- WO-A1-01/25789
- WO-A1-2005/066356
- WO-A1-2008/044530
- US-A1- 2005 123 443
- US-A1- 2005 183 494
- CHEN J ET AL: "Reagentless amperometric immunosensor for human chorionic gonadotrophin based on direct electrochemistry of horseradish peroxidase" BIOSENSORS AND BIOELECTRONICS, ELSEVIER BV, NL LNKD- DOI:10.1016/J.BIOS.2004.10.023, vol. 21, no. 2, 15 August 2005 (2005-08-15), pages 330-336, XP004981157 ISSN: 0956-5663

## Description

The present invention relates to easy to use diagnostic test devices, particularly suited for use by people at home, and by doctors and nurses in a primary or home healthcare setting. An example is a convenient and easy to use immunoassay test for pregnancy using a urine sample.

The use of immunoassay test devices for the detection of pregnancy is commonplace. Test devices to date have generally relied on the optical interpretation of a coloured or other visual end point, for example the development of pink or blue stripes or spots on a flat white membrane. The vast majority of devices proposed to date require the visual determination of the coloured end point using the eye. Examples of such "read by eye" test devices are described in US Patent 6,352,862 and US Patent 6,485,982. End point detection by an instrumental optical means has been proposed and a number of immunoassay test products to date have employed such means, for example by the incorporation of reflectance photometry; an example of such a test device is described in US Patent 5,580,794.

Visual detection means in diagnostic tests are prone to misinterpretation by the user and cannot provide accurate and precise quantitative test results. Early glucose tests, for the management of diabetes, employed visual interpretation of colour developed on the surface of a test strip. The colour on the test strip was compared to a printed colour scale; the user would match the colour on the test strip to a corresponding printed colour and in turn read a corresponding glucose concentration from a range printed on the colour scale. To improve accuracy and reliability of interpretation, glucose tests were modified subsequently by the provision of an instrument which typically contained a reflectance photometer to read the colour of the test strip by instrumental means, thus reducing the potential for misinterpretation by the user. While reflectance photometry is an improvement over determination by the human eye, there remain limitations of accuracy and reliability inherent in optical detection. For this reason, monitoring tests for diabetes have been improved further by the conversion of glucose test systems from a visual or optical end point to an electrochemical end point, facilitated by the adoption of various electrochemical biosensor techniques. In recent years, electrochemical biosensor based devices have become the preferred means of glucose measurement for the self-monitoring of diabetes because they provide accurate and precise quantitative test results.

However, sensors based on detecting an electrochemical end point can be more complex than sensors which rely on optical endpoints and may be less easy to use, particularly for people at home or by doctors and nurses in a primary or home healthcare setting. Such sensors may also be more expensive to manufacture and more complex to incorporate into a diagnostic test device.

WO 2005/066356 A1 discloses a non-mediated biosensor for indicating amperometrically the catalytic activity of an oxidoreductase enzyme in the presence of a fluid containing a substance acted upon by said enzyme comprising: (a) a first substrate; (b) a working electrode and a reference electrode on the first substrate; (c) conductive tracks connected to said electrodes for making electrical connections with a test meter apparatus; (d) a second substrate overlying part of the first substrate; and (e) a spacer layer having a channel therein and disposed between the first substrate and the second substrate, the spacer layer channel co-operating with adjacent surfaces to define a capillary flow path which does not contain a mesh and which extends from an edge of at least one of said substrates to said electrodes.

WO 2008/044530 A1 discloses a multicomponent analysis sensor for measuring two or more kinds of the subjects to be measured by using redox reactions, which is a multicomponent analysis sensor comprising a liquid sample inlet from which a liquid sample containing two or more kinds of the subjects to be measured is introduced, a first measurement chamber, a second measurement chamber, a first channel connecting the above-described liquid sample inlet to the above-described first measurement chamber and a second channel connecting the above-described first measurement chamber to the second measurement chamber, wherein the above-described first measurement chamber and the above-described second measurement chamber respectively have a working electrode and a counter electrode. A first reagent layer containing an enzyme and an electron transfer substance is provided in the first channel or the first chamber, while another reagent layer containing an enzyme is provided in the second channel or the second chamber.

US 2005/0183494 A1 discloses a device having a flow channel, wherein a layer of mesh is adhered to a surface forming a wall of a flow channel, but the layer of mesh is of such dimensions that the layer of mesh does not contact those portions of the device where electrochemical reactions occur and electrons flow. In one aspect, the invention provides a sensor, such as, for example, a biosensor, in the form of a strip, the sensor being suitable for electrochemical or optical measurement. The sensor comprises a base layer and a cover layer having a layer of mesh adhered thereto, and the base layer is separated from the cover layer by a spacer layer. The base layer, the cover layer having a layer of mesh adhered thereto, and the spacer layer define a flow channel into which a liquid sample is drawn therein and flows therethrough by means of wicking.

EP 1 712 919 A1 discloses an analytical instrument which includes a flow path for moving a sample containing blood cells, an introduction port for introducing the sample into the flow path, a reagent portion arranged in the flow path, and an electron detection medium for obtaining information necessary for analyzing an analysis target component contained in the sample in relation with an amount of electrons transferred. The reagent portion contains an electron mediator for supplying an electron taken from the analysis target component in the sample to the electron detection medium, and at least part of the reagent portion is positioned adjacent to the introduction port.

US 2005/0123443 A1 discloses a sensor for blood component analysis, which allows even a trace amount of blood to be led to an analysis portion reliably. The sensor for blood component analysis includes a substrate, a spacer, and a cover. The cover is disposed on the substrate with the spacer intervening between the cover and the substrate, whereby a space that serves as an analysis portion and a channel for leading blood to the analysis portion is formed inside the sensor.

CHEN J ET AL: "Reagentless amperometric immunosensor for human chorionic gonadotrophin based on direct electrochemistry of horseradish peroxidise"

BIOSENSORS AND BIOELECTRONICS, ELSEVIER BV, NL LNKD - DOI: 10.1016/J.BOIS.2004.10.023, vol. 21, no, 2 15 August 2005 (2005-08-15), pages 330-336, XP004981157 ISSN: 0956-5663 discloses an immunosensor for determination of HCG implemented by immobilisation of HCG with titania sol-gel on a glassy carbon electrode and the direct electrochemistry of HRP labelled to HCG antibody.

EP 1 443 327 A2 discloses an assay device which comprises a substrate comprising (i) a porous material capable of chromatographically transporting a liquid and (ii) one or more test reagents for an assay provided on the porous material; and a doping polymer impermeable to the liquid which the porous material is capable of transporting, incorporated into a part of the porous material so as to define a channel in the porous material, the one or more test reagents being provided on the porous material in the channel defined therein.

WO 01/25789 A1 discloses an assay device for an analyte comprising a flow path for a liquid defined by interstices in a porous medium, at least one liquid flow barrier which is impregnated into and immobilised on the porous medium at a location in the flow path, at least one barrier release means which is impregnated into the porous medium, and an analyte capture region in the flow path. The barrier release means is soluble in the liquid, and, in use, is moveable by liquid flowing in the flow path into contact with the flow barrier so as to permit liquid flow through said location. Preferably, an analyte visualising agent is provided at a zone in the flow path, said visualising agent being capable of interacting with the analyte to indicate the presence of the analyte.

It is an object of the present invention to provide a sensor that at least partially overcomes at least one of the problems mentioned above in relation to the prior art.

According to an aspect of the invention, there is provided an assay module for an electrochemical test device, said assay module comprising: at least one channel for transporting a liquid to be tested from a deposition zone, where a sample of said liquid to be tested can be deposited, to a testing zone, the testing zone being spaced apart from said deposition zone; and electrodes for measuring an electrical property of liquid in said testing zone (6), characterized in that: said at least one channel comprises a porous material capable of transporting said liquid chromatographically; and the assay module further comprises a polymer that is impermeable to the liquid which the porous material is capable of transporting, incorporated into a part of the porous material so as to define a channel in the body of the porous material, the deposition zone and testing zone being provided on the porous material in the channel defined therein.

The assay module thus defined is inexpensive to manufacture and easy to use. For many applications, users will be able to apply samples in the same (or a very similar) way as would be necessary with prior art devices relying on optical endpoints. In contrast to such optical endpoint devices, however, the assay module of the present invention allows for detailed quantitative determination of analyte concentrations. The inaccuracies associated with optical endpoint devices are not present. The results of measurements can be presented in a wide variety of formats that do not rely on interpretation of visual endpoints. Use of the device is thus safer and more reliable. Furthermore, this can be achieved without having to send samples to external laboratories for detailed analysis. The device is thus ideal for use by people at home or by doctors and nurses in a primary or home healthcare setting.

According to a further aspect of the invention, there is provided an electrochemical test device, comprising an assay module according to an embodiment of the invention and means for receiving an assay module according to an embodiment of the invention; and an analysis and display module comprising: a measurement section for interacting with said electrodes in said assay module in order to measure an electrical property of liquid in said testing zone; an analysis section for obtaining information about the composition of said liquid to be tested based on said electrical property of liquid in said testing zone measured by said measurement section; and a display section for displaying said information obtained by said analysis section.

According to a further aspect of the invention, there is provided a method of testing a liquid sample to determine information about the composition of said liquid sample using an electrochemical test device according to an embodiment of the invention, said method comprising: depositing said liquid sample in said deposition zone; and reading information displayed on said display section.

Embodiments of the invention will now be described, by way of example only, with reference to the accompanying schematic drawings in which corresponding reference symbols indicate corresponding parts, and in which:
Figure 1 shows an assay module according to an embodiment of the present invention;
Figures 2A to 2F show various configurations of channels in an assay module;
Figure 3A shows an example complex, comprising a detectable label conjugated to a specific binder that is itself bound to an analyte; Figure 3B shows an unbound specific binder for an analyte, immobilized at a testing zone;
Figure 4 illustrates a flow of a fluid sample containing a target substance to be detected in an assay, together with an added antibody-enzyme conjugate specific to the target substance, through an assay module containing an immobilised capture antibody also specific to the target substance; the target substance may bind to either or both of the conjugate and capture antibody;
Figure 5 illustrates a flow of a fluid sample that does not contain a target substance, together with added antibody-enzyme conjugate specific to a target substance, through an assay module containing an immobilised capture antibody also specific to a target substance; the absence of a corresponding target substance means no specific binding is possible and therefore the conjugate moves through the assay module without becoming bound to a target substance or to a complex of target substance bound to capture antibody;
Figure 6A to 6C illustrate example reactions for implementing the electrochemical test device by detecting the enzyme present in a conjugate;
Figure 7 shows an analysis and display module configured to receive a detachable assay module;
Figure 8 shows an analysis and display module with integral assay module;
Figure 9 illustrates various components of an analysis and display module;
Figure 10 is a cross-section view along A-A of the assay module shown in Figure 1;
Figure 11 depicts a reaction scheme for the assay of glucose using glucose oxidase and horseradish peroxidase (HRP) with the electrochemical detection of oxidised ABTS;
Figure 12 depicts an example electrode configuration;
Figures 13A and 13B show experimental results of current response of Fusion 5 paper - GOx electrodes to glucose;
Figure 14 shows an alternative embodiment of the assay module;
Figure 15A and 15B show experimental results using the assay module of Figure 14; and
Figure 16 shows the reaction scheme used for performing electrochemical determination of HRP in order to obtain the results shown in Figures 15A and 15B.

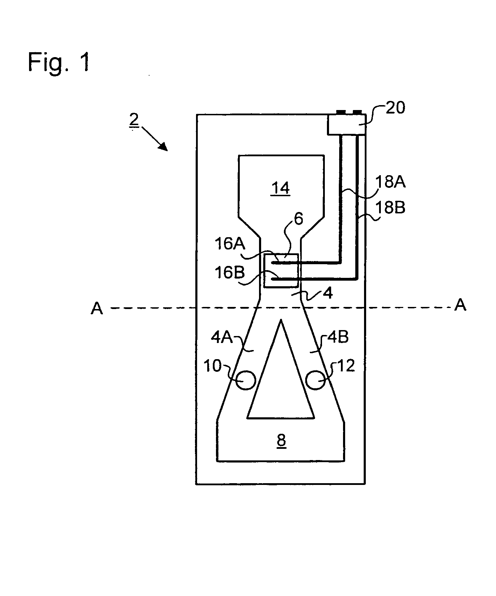

Figure 1 shows an assay module 2 according to an embodiment of the present invention. Channels 4, 4A and 4B are formed within the assay module 2 for containing a flow of a liquid to be tested from a deposition zone 8, where a user deposits the liquid, to a testing zone 6 where the liquid is analysed. The flow path is arranged to pass via one or more reagents 10 and 12, which are initially localized at positions between the deposition zone 8 and testing zone 6 in channels 4A and 4B. The one or more reagents 10 and 12 may comprise test reagents that are suitable for the specific determination of the presence of one or more substances in the liquid sample, and may also comprise detection reagents that facilitate detection or measurement of the presence of one or more substances in the sample by an electrochemical means. The test reagents may, for example, comprise an antibody, antigen, receptor or other binder for specifically binding to a target substance for the purpose of its detection or measurement. Examples of possible detection reagents are given below. In the embodiment shown, the channel 4A contains test reagent and may be referred to as a "test reagent channel", while the channel 4B contains detection reagent and may be referred to as a "detection reagent channel". In general, the test and detection reagents will be provided in a form that is localized initially (e.g. a dry form), but which becomes mobile on arrival of the liquid, such that the reagents are carried along with the liquid. A waste channel 14 and optional waste sink or reservoir may be provided for receiving liquid after it has passed through the testing zone 6.

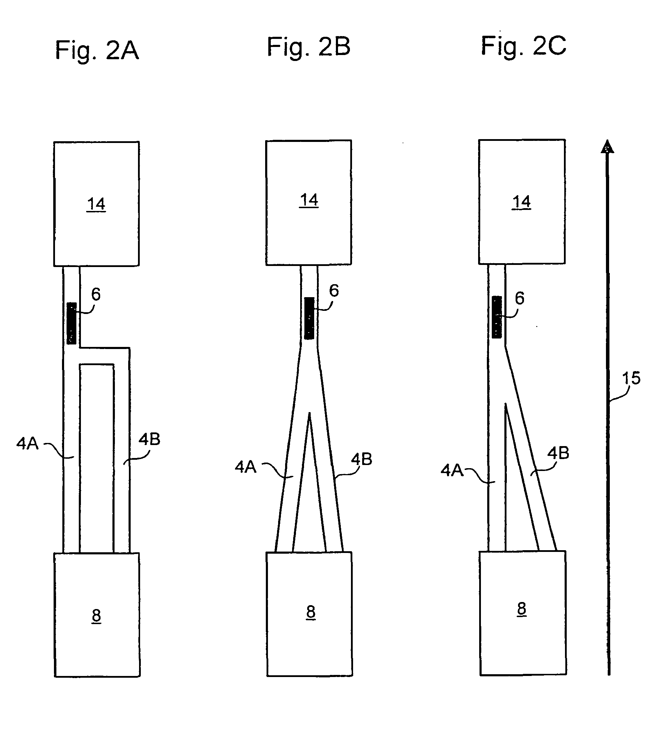

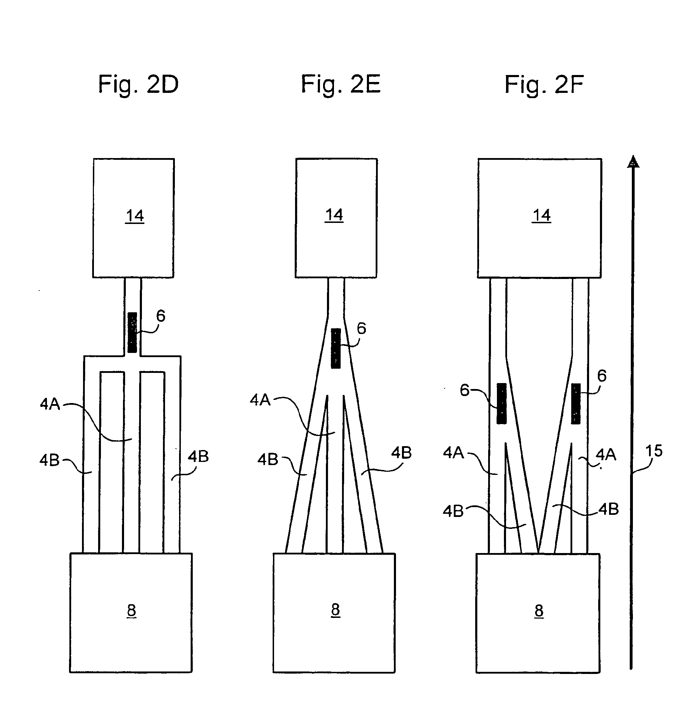

Figures 2A to 2F show various possible channel and testing zone configurations for the assay module 2. Each arrangement is configured such that liquid added at the deposition zone 8 will flow through the test and detection reagent channels 4A and 4B to one or more testing zones 6, and then to a waste channel 14. Figures 2A to 2C show examples comprising one test reagent channel 4A and one detection reagent channel 4B, leading to a single testing zone 6. In Figures 2A and 2C, the detection reagent channel 4B is longer than the test reagent channel 4A, which may be useful for ensuring that detection reagents reach the testing zone 6 after arrival of test reagents. In Figure 2B, the test and detection reagent channels 4A and 4B have the same length; the test and detection reagents may therefore arrive at the testing zone 6 simultaneously. Alternatively the detection reagent channel 4B may be modified to cause the liquid to propagate along it more slowly than along the test reagent channel, so that the detection reagents arrive at the testing zone 6 after the test reagents. Figures 2D and 2E depict arrangements having one test reagent channel 4A and two detection reagent channels 4B. The detection reagent channels 4B are longer than the test reagent channel 4A in each case.

Figure 2F shows an arrangement for detecting two different analytes via separate testing zones 6. The assay module 2 according to this embodiment has two pairs of channels, each pair consisting of a test reagent channel 4A and a detection reagent channel 4B leading to one of the two testing zones 6. In the embodiment shown, in each of the pairs of channels, the detection reagent channel 4B is longer than the test reagent channel 4A.

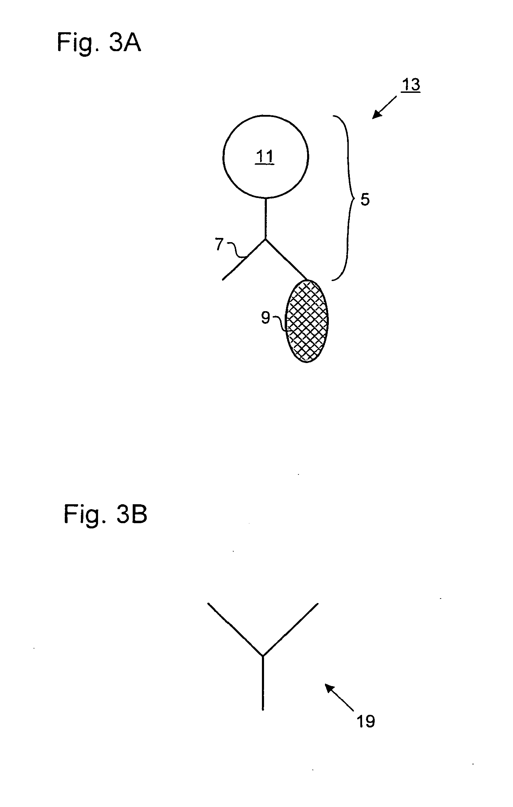

Figure 3A depicts a complex 13 that may be used with embodiments of the present invention. Here, an enzyme label 11 is used, this label being conjugated to a specific binder 7, such as an antibody specific for a target substance 9 ("analyte") to be detected or measured in a sample. In this embodiment, the enzyme-antibody conjugate 5 would be immobilised or otherwise bound or captured at the testing zone 6 if the target substance 9 is present in the sample, by forming a complex 13 with the conjugate 5. This could be facilitated by the incorporation of at least a further second specific binder 19 immobilised or trapped at the testing zone 6 (Figure 3B). This further binder 19 at the testing zone 6 may be either immobilised directly (for example by chemical bonding or physical adsorption) at the testing zone 6 or may be captured or otherwise immobilised via a biochemical means (for example via a streptavidin-biotin coupling interaction) or some other means. The presence of bound enzyme label 11 could be determined electrochemically by coupling a reaction between the enzyme 11 and one or more appropriate enzyme substrates to an electrode. Such a testing zone 6 may be referred to as an electrochemical biosensor. The electrochemical reaction may optionally be facilitated by the use of an electrochemically active mediator substance present in the testing zone. For example, if the enzyme label were peroxidase, the label could be detected by adding hydrogen peroxide, or a source of hydrogen peroxide, to the testing zone 6 and coupling or otherwise relating the reaction of the peroxidase and the hydrogen peroxide via a mediator substance, such as TMb or ABTS, to an electrode printed or otherwise present at the testing zone 6.

Alternative detection reactions using different enzymes and substrates are possible. For example, a preferred embodiment of the present invention might incorporate glucose oxidase as the enzyme and glucose as the substrate and their reaction could be either coupled directly to an electrode which detects hydrogen peroxide produced by the reaction or coupled to a second enzyme, for example peroxidase, in a reaction mixture which consumes hydrogen peroxide, this consumption being measured at an electrode using a mediator substance which participates in the reaction scheme. Alternatively, the glucose oxidase reaction could be coupled to an electrode using a mediator such as ferrocene or a derivative of ferrocene.

More generally, liquid sample travelling from the deposition zone 8 to the testing zone 6 will mobilize a test reagent 10 in a test reagent channel 4A, forming complex 13 if analyte 9 is present. Complex 13 will be carried to the test zone 6 where it may be immobilized by binding to a specific binder 19, thereby changing the electrical properties of the liquid between the electrodes 16A and 16B in the testing zone 6 due to the presence of the detectable label 11 and/or detection reagents. The electrodes 16A and 16B can be used to measure the change in electrical properties and the concentration or simply the presence of the analyte present in the sample under test can be deduced from the measurements. The test system may be arranged, for example, such that the degree of binding at the testing zone 6 is proportional to the concentration of analyte 9 in the sample.

Figure 3B illustrates an example where no specific binding of conjugate 5 has occurred at the testing zone 6 and the binding sites of the specific binder 19 immobilised at the testing zone 6 are not occupied by analyte 9 bound to conjugate 5. In some instances, the immobilised specific binder 19 may be bound to analyte 9 that is not bound to conjugate 5 (not shown). In instances where there is no conjugate 5 specifically bound at the testing zone 6, no useful electrochemical signal may be detected. Under normal test conditions, it is possible that conjugate 5 may become bound non-specifically at the testing zone 6 (not shown). In an optimised test system, such non-specific binding may occur to a limited extent that would not give rise to an incorrect test result and it is possible optionally to include a negative control means where non-specific binding may be estimated and corrected for when a test result is determined.

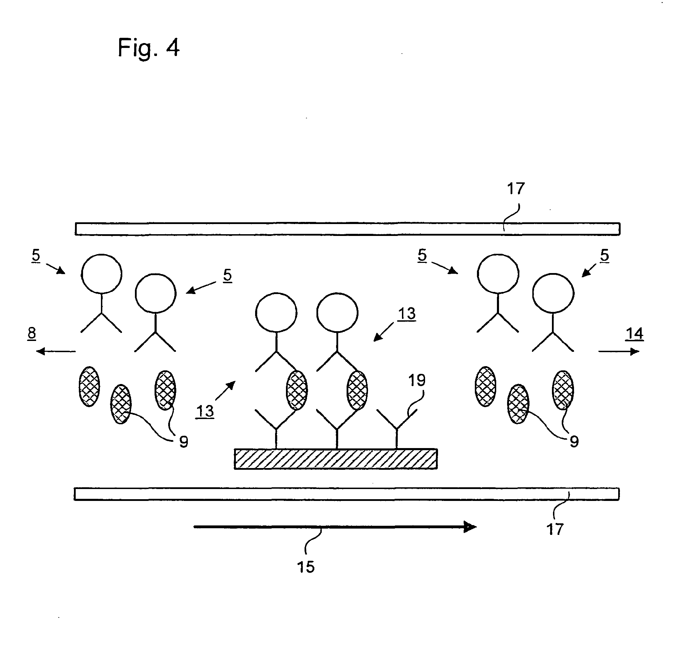

Figure 4 illustrates the process of a liquid sample flowing from the deposition zone 8 to the sample zone 6 and sink 14. Mobilized conjugate 5 and analyte 9 are present upstream of the testing zone 6. At the testing zone 6, the conjugate 5 can become bound to the specific binder 19 immobilized in the testing zone 6 if it has first become bound to the analyte 9. Conjugate 5 that does not bind at the testing zone 6 may be washed from the testing zone 6 and into the waste channel 14. The channels 4, 4A and 4B and the testing zone 6 may be defined by one or more boundaries 17. Some of the conjugate 5 washed from testing zone 6 may be bound to analyte 9 from the sample only (not shown). The liquid flow within the channels 4, 4A and 4B is indicated by an arrow 15.

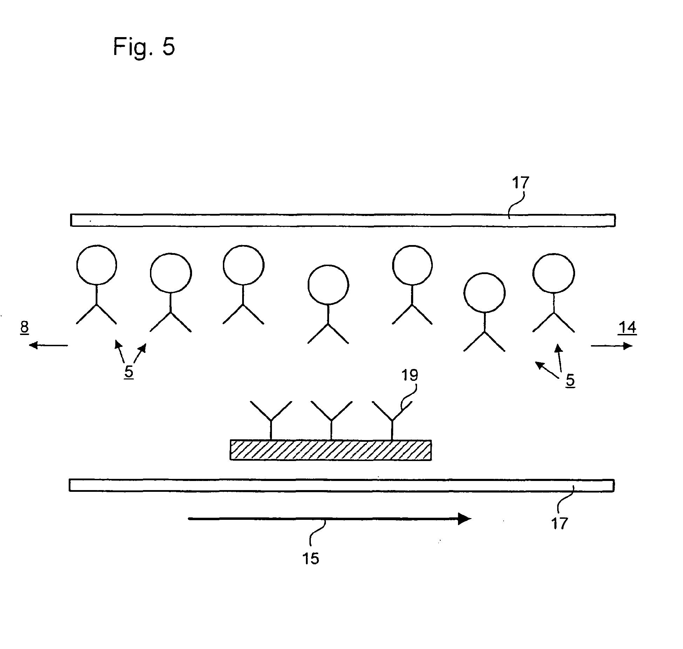

Figure 5 illustrates the nature of the absence of binding of test reagents at the testing zone 6, which gives rise to no useful electrochemical signal being detectable if the test system is of the commonly used "sandwich" type. Lack of binding may result from there being no analyte in a sample or there being a relatively low concentration of analyte in a sample. In a balanced test system the amount of unbound test reagents will increase proportionally as the concentration of analyte in a sample decreases.

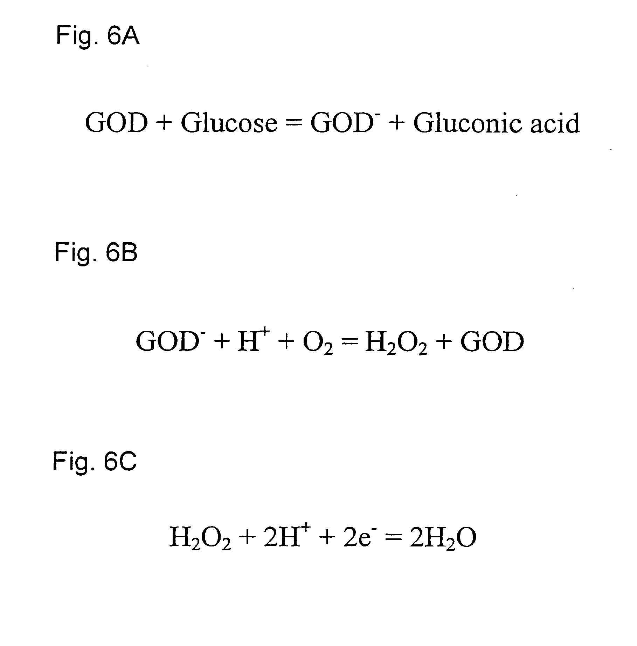

Figures 6A, 6B and 6C illustrate by way of example a biochemical reaction that could be used as the basis for electrochemical detection of an analyte. In this example, the conjugate 5 comprises the enzyme glucose oxidase (GOD) with glucose as a substrate used in the detection reaction. The GOD converts the glucose to gluconic acid and in the process becomes reduced (GOD⁻) (Figure 6A). The GOD⁻ reacts with hydrogen ions (H⁺) and oxygen (O₂) present in the reaction mixture and hydrogen peroxide (H₂O₂) is produced (Figure 6B), which may subsequently be detected electrochemically by virtue of a reaction at an electrode (16A, 16B) in which the hydrogen peroxide is caused to react with hydrogen ions (H⁺) and electrons (e⁻) to produce water (H₂O) (Figure 6C). The electrons participating in the overall reaction scheme interact with the electrode system (16A,16B) present at a testing zone 6 in the assay module 2 to produce a detectable electrical signal which may be measured amperometrically.

Detection reagents may be used to facilitate electrochemical detection of bound conjugate at the testing zone 6. For example, if the enzyme 11 to be bound at the testing zone 6 is peroxidase, as in a pregnancy test, then the detection reagents will include hydrogen peroxidase or alternatively a chemical mixture that will produce hydrogen peroxide at an appropriate time or stage in the running of a test. A mediator substance such as TMB or ABTS may also be present. The test and detection reagent channels, 4A and 4B respectively, are configured within the assay module 2 such that a liquid can enter the detection reagent channel 4B and hydrate the detection reagents 12, making them mobile in liquid within the detection reagent channels 4B. This liquid flows in a direction towards the testing zone 6 such that the flow will deliver the detection reagents 12 to the testing zone 6 at an appropriate stage in the test procedure. Specifically, detection reagents 12 are delivered to the testing zone 6 after unbound conjugate 5 has been washed away from the testing zone 6. This is facilitated by configuring the assay module 2 such that liquid in a detection reagent channel 4B flows into a test reagent channel 4A upstream of the testing zone 6, such that the detection reagents 12 flow into the testing zone 6 in the same direction as the flow path taken by the test reagents 10. Figures 2A to 2F illustrate such arrangements.

In the hCG test embodiment described below, the test reagent channels 4A and detection reagent channels 4B may be connected to a common sample reservoir which collects the added urine sample and supplies this liquid to each channel. Figures 2A to 2E illustrate such arrangements. Alternatively, the channels 4A and 4B may be fed liquid from separate collectors or reservoirs, facilitating the use of a specially formulated detection reagent buffer as an alternative to the use of the liquid sample within the detection reagent channel 4B.

A number of mechanisms may be employed to ensure that the detection reagents 12 arrive at the testing zone 6 at the correct time, i.e. after unbound conjugate reagent 5 has been removed from the testing zone 6 by the progressing liquid flow present in the channels. The length of the detection reagent channel 4B could simply be made longer than that of the test reagent channel 4A upstream of the testing zone 6, so that liquid in the detection reagent channel 4B will take more time to reach the testing zone 6. It is also possible that substances could be deposited in the detection reagent channel 4B to slow down the flow of liquid along this channel 4B. Such substances could include without limitation salts, waxes, polymers, particulate materials and gels. Additionally, the test and detection reagent channels 4A and 4B could be selectively shaped to control the flow of liquid in the channels as desired.

Within a single assay module 2 according to an embodiment, multiple test reagent channels 4A may be present. Each of the multiple test reagent channels 4A may be provided with a corresponding separate testing zone 6 and waste channel 14, with one or more optional detection reagent channels 4B. The incorporation of multiple channels facilitates the measurement of different analytes 9 in a single test, or the measurement of single analyte 9 more than once using the same sample, to improve test precision and reliability. Incorporating more than one test reagent channel 4A would also allow the assay module 2 to include one or more test reagent channels 4A that can perform the function of negative control channels. Such a negative control channel could, for example, include test reagents 10 and detection reagents 12, but with the test reagents 10 formulated so that no specific immobilisation of analyte 9 can occur. In this formulation, any immobilisation of antibody-enzyme conjugate 5 taking place at the control zone (which is similar or otherwise identical in construction and function to the testing zone 6 for detecting analyte) will be due to non-specific binding effects (NSB). NSB is a particular and well known problem in all immunoassays and the estimation or measurement of NSB can improve test sensitivity, reliability and precision. Such a control channel within an assay device can also act as a procedural control, performing a similar control function as the control stripe or spot of a conventional lateral flow test, indicating that the test device 1 is functioning properly. Optionally, control channels could also be configured to act as test calibration or positive control channels.

Where there is no optional detection reagent channel 4B in the assay module 2, detection reagents 12 for the electrochemical measurement of conjugate 5 bound at a testing zone 6 may be added to the testing zone 6 by some means other then a detection reagent channel 4B. For example, an operator or technician may add detection reagents 12 to the testing zone 6 at an appropriate time during the performing of a test, using a dropper or a pipette. Alternatively or additionally, detection reagents 12 could be added by a mechanism incorporated into an analysis and display module 26 (see below), which could control and direct the addition of detection reagents at an appropriate time in the performing of a test. Such a modified analysis and display module 26 may optionally house detection reagents using a suitable containment means. A modified analysis and display module 26 as described herein may be referred to as an "analyser" or "Point of Care" instrument.

Biosensor electrodes 16A and 16B are provided in the testing zone 6, and conductive tracks 18A and 18B provide electrical connection to an electrical interface 20 of the assay module 2. Optionally, suitable additional electronics (for example a means of applying a defined electrical potential to the biosensor electrodes) may be incorporated within the testing zone 6 and/or assay module 2 to facilitate the performing of an electrochemical measurement. The configuration and particular construction of the electrodes 16A and 16B (or single electrode - not shown) may be governed by the particular nature of the electrochemical detection technique to be used within the assay module 2. For example, reagents for the electrochemical reaction may be included in the composition of the electrodes 16A and 16B. It may often be convenient to form electrodes 16A and 16B (and/or conductive tracks 18A and 18B) by printing or otherwise depositing conductive inks or paints onto a substrate of the assay module 2. One or more of the reagents for the electrochemical reaction in question may thus be included in the conductive ink composition. More generally, the conductive inks or paints may comprise carbon, silver or platinum, for example.

Alternatively, electrodes 16A and 16B may be comprised of films, bonded or otherwise fixed in place at the testing zone 6. It may also be possible to add a third element to the electrode system, such as a reference electrode.

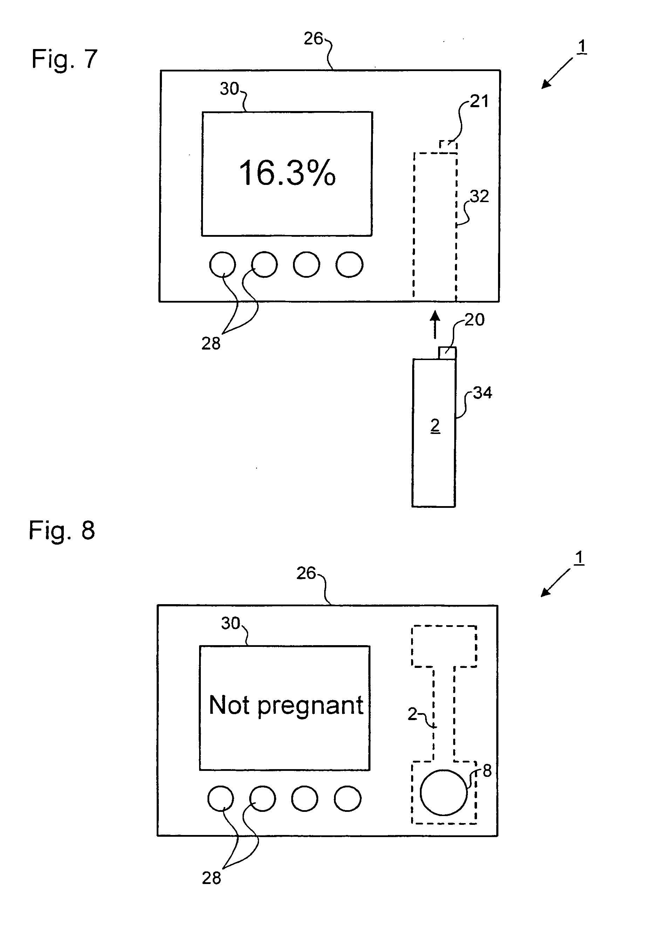

Figure 7 depicts an embodiment of the complete electrochemical test device 1. The device 1 comprises an analysis and display module 26 and a detachable assay module 2. The analysis and display module 26 has a mechanical interface 32 for receiving the assay module 2 via a corresponding mechanical interface 34 on the assay module 2. The respective interfaces 32 and 34 may be arranged to ensure correct alignment of the assay module 2 within the analysis and display module 26 and/or to encourage correct electrical connection and/or to discourage inadvertent mechanical and/or electrical disconnection during use. The assay module 2 comprises an electrical interface 20 that is configured to engage with a corresponding electrical interface 21 within the analysis and display module 26. The respective electrical interfaces 20 and 21 allow connection between measurement electronics within the analysis and display module 26 and electrodes 16A and 16B within the testing zone 6 in the assay module 2 (see Figure 1).

A display 30 is provided for communicating the results of an analysis of a liquid sample deposited on the assay module 2. The display may be configured to communicate the results in the form of letters and/or numbers. For a given component analyte 9 within the liquid sample, the analysis and display module 26 may be arranged to show at least three different images (for example, "low", "medium" and "high", or "10%", "50%" and "80%") corresponding to three different detected concentrations or three different ranges of compositions. The display 30 can therefore take advantage of the possibility of quantitative or semi-quantitative measurements using the electrochemical approach of the present invention. In addition, this enhanced flexibility of the display 30 promotes more intuitive interpretation of the results, compared with systems using symbols (such as lines or spots) to communicate a binary positive or negative result. The risk of erroneous interpretation is therefore reduced.

User input means 28 (for example, buttons or switches) may be provided for controlling what the display 30 displays. For example, the user input means 28 may be configured to switch the display 30 from a state in which it is displaying information about the composition of a first sample to a state in which it is displaying information about the composition of a second sample, different from said first sample. Alternatively or additionally, the user input means 28 may be configured to switch the format of the display, for example to change the number of significant figures or decimal points shown, to switch between a digital and analogue display, to add or remove a display of a measurement "error" or accuracy indication, and/or to control the units that are displayed.

The format of the test result may be selected automatically, or pre-selected, according to the specific intended application of the test device 1. A pregnancy test, for example, may require that the test result is in a "yes/no" binary format. Many infectious disease tests, tests for drugs of abuse and other tests may also require test results in a "yes/no" format. For these applications the analysis and display module 26 may be configured (via software and a microprocessor, for example) to provide test results as "yes" or "no".

In diagnostic or other analytical applications which require quantitative or semi-quantitative test results, for example in point of care tests for use by healthcare professionals, the test device 1 could be configured to provide a quantitative or semi-quantitative test result format. For example, a test for luteinising hormone (LH) might yield test results using the semi-quantitative format "low", "medium" or "high". Current self-use tests for LH, intended for predicting ovulation in women, are "yes/no" tests and are identical in form and in use to pregnancy tests with the exception of the specificity of the test chemistry employed. The facility to report measured LH levels as "low", "medium" or "high" could be advantageous in the prediction of ovulation in both the home and the point of care setting. Quantitative reporting of LH results may also be possible with the present invention, with test results expressed as standardised units. In an LH test, the preferred units would be mIU/ml, or "milli International Units per millilitre". Embodiments of the present invention that produce quantitative test results could have widespread application in human and veterinary healthcare and also in other analytical fields, including environmental testing.

The display 30 may be an electronic display, which can consist of a variety of optional electronic display means, ranging from simple light emitting diodes (LEDs) or other simple optical indicators to alphanumeric displays, which display numbers and/or text. In a preferred embodiment of the present invention, the display 30 is configured to display a text message to indicate the test result in a yes/no format in the form of a textual message. For example, in a test device for pregnancy, this message might simply read "pregnant" or "not pregnant" and in a test device for ovulation detection, the message might read "ovulating" or "not ovulating". The same display format could be used to indicate the presence or absence of target substances. For example a drugs of abuse test device might display "No drugs present" or "Cocaine present". Simplified embodiments of the present invention might incorporate simple LEDs which by either being lit or not lit, or displaying one colour or another (such as red or green) could impart the test result to the user in a yes/no format.

In an embodiment where the test device is intended to provide a semi-quantitative test result, the electronic display 30 could be an alphanumeric display and could be configured to display messages in a semi-quantitative format, for example "low", "medium" or "high", or similar, as appropriate. Embodiments of the present invention might use a number of simple LEDs to indicate semi-quantitative results, for example yellow, green and red LEDs, where the colours correspond, for example, to low, medium and high concentration respectively. Alternatively, a number of LEDs of, for example, a single colour could be illuminated in series such that an increased number of illuminated LEDs indicates an increased concentration of analyte.

In an embodiment where the test device is intended to provide a quantitative test result, the display 30 could be an alphanumeric display configured to display messages in a quantitative format. For example, concentration could be expressed as a number followed by appropriate units, for example 2.3 mmol/L (millimoles per litre) or 23.5 mIU/ml. Embodiments of the present invention which produce quantitative test results could have widespread application in human and veterinary healthcare and also in other analytical fields, including environmental testing.

In arrangements in which the assay module 2 is detachably connected to the analysis and display section 26, the assay module 2 may be disposable (i.e. configured to be used a limited number of times, for example once, and then disposed of), while the analysis and display section 26 is retained for a large number of uses. This arrangement may make it easier to ensure condition of the assay modules 2 as they could be provided in sterilized or sealed packs and only opened when they need to be used. In addition, the electrochemical test device 1 can easily and economically be adapted to different types of test by simply changing the nature of the assay module 2; the analyis and display module 26 need not be altered. The sample may be added to the assay module 2 before or after its attachment to the analysis and display section 26. After use, the assay module 2 may be ejected or removed from the electrochemical test device 1.

Figure 8 illustrates an alternative arrangement in which an assay module 2 is integral (i.e. not removable in whole or in part) within the analysis and display module 26, in such a way that the whole electrochemical test device 1 is intended for one use only, thereafter to be discarded.

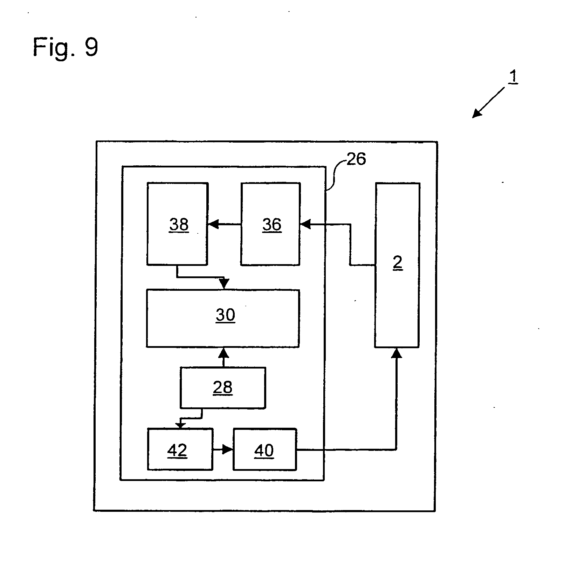

Figure 9 shows the operational elements of an embodiment of the electrochemical test device 1. As mentioned above, the electrochemical test device comprises an integral or detachable assay module 2 and an analysis and display module 26. The assay module 2 is electrically connected to the analysis and display module 26 such that a measurement section 36 can measure an electrical property of liquid in the testing zone 6 in the assay module 2. For example, the measurement section 36 may be configured to apply a fixed voltage across the electrodes 16A and 16B in the testing zone 6 and measure the resulting electrical current. Alternatively, the measurement section 36 may be configured to apply a fixed current between the electrodes 16A and 16B and measure the resulting voltage (i.e. the voltage necessary to maintain that current). Alternatively, an alternating voltage or current could be applied and the resulting current or voltage measured (for example, using phase sensitive detection). The measurement section 36 may be configured to measure the capacitance of the electrode system 16A, 16B and/or the resistivity of the material between the electrodes 16A and 16B.

The measurement system 36 may be provided with an integral power source, for example a battery, photoelectric means, inductance means, or a "wind-up" mechanism. Alternatively or additionally, the power source may be provided within the assay module 2. Alternatively or additionally, the test device 1 may be configured to be connected to an external power source, such as a mains source.

The electrical properties (e.g. current, voltage, resistivity, capacitance, etc.) measured by the measurement section 36 may be forwarded to an analysis section 38 within the analysis and display module 26. The analysis section 38 may be implemented by suitable processing hardware, including for example one or more CPUs, memory devices etc. along with suitable software for controlling the processing hardware. The analysis section 38 is configured to interpret the measured electrical properties in order to determine the concentrations of the one or more analytes targeted by the electrochemical test device 1. A memory containing calibration tables and an appropriate interpolation scheme implemented by the software may be used for this purpose.

Generally, there should be a sufficient amount of liquid sample added to the assay module 2 to facilitate the transfer of the sample and reagents throughout the channels 4 of the assay module 2, by capillary or chromatographic flow for example. A sufficient volume of liquid may be incorporated into the assay module 2 by adding the sample to a sample deposition zone 8, for example. Alternatively or additionally, the sample deposition zone 8 may be dipped into a liquid sample. A urine sample would normally provide a sufficient volume to facilitate the running of a test and can be added to the deposition zone 8 or incorporated by dipping the deposition zone 8 into a sample held in a suitable receptacle. These two means of sample addition are common in commercial pregnancy and ovulation tests.

Alternatively, the sample volume may not be sufficient to facilitate the performing of a test. For example, the sample may be a drop or similarly small volume of pre-treated or untreated serum, plasma or whole blood. Such a small volume of sample could be added to deposition zone 8 upstream of the test reagents and means provided for a suitably formulated buffer solution to be added further upstream to facilitate movement of the sample throughout the assay module 2 to facilitate performance of a test. Such a buffer solution could be added to a sink or reservoir located within the assay module 2 or the assay module 2 could be dipped into the buffer solution.

In a preferred embodiment of the present invention, which would be applicable for example but not by limitation to Point of Care testing by healthcare professionals, the analysis and display module 26 could take the form of a test processor or analyser which would perform the functionality of an analysis and display module 26 and additionally incorporate a suitable liquid reagent (for example, a buffer) and a means of adding this reagent to the assay module 2 at an appropriate volume and with appropriate timing so as to facilitate the performing of a test. Assay modules 2 for use with such a modified analysis and display module 26 would typically be disposable, while the modified analysis and display module 26 would typically be re-usable to allow many tests to be performed.

Figure 9 illustrates one possible arrangement. A reservoir 40, encased by a water impermeable material, such as a plastic, ceramic or a metallic foil, is provided for containing a liquid reagent (such as a buffer) to be added to a channel 4 in the assay module 2 to facilitate performance of a test. The liquid reagent may be added via a release mechanism 42, which may be automatically controlled (for example so as to trigger release of the liquid at an appropriate time for a given test procedure, such as shortly after a liquid sample has been deposited in the deposition zone 8 by a user) or manually controlled (as shown) via the user input section 28. The reservoir and/or release mechanism may form part of the analysis and display module 26 (as shown) and/or of the assay module 2. Pumps, valves, nozzles or other means of manipulating a flow of liquid may be provided as necessary to implement the required functionality.

The results of analysis by the analysis section 38 can be forwarded to the display 30 for display as described above.

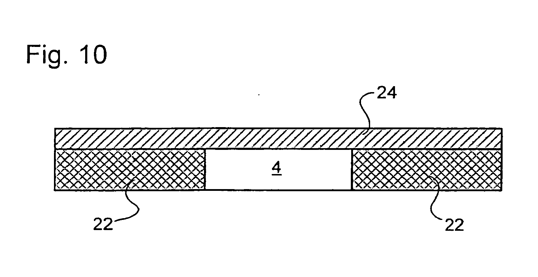

Figure 10 illustrates a cross-sectional view along line A-A of Figure 1. This view shows how the assay module is constructed using a polymer modified absorbent or porous material according to an embodiment of the invention. The process of polymer modification is described in WO09832018A1. The absorbent/porous material is generally flat and may be supplied in sheet form or in roll form. This material may typically be either a cellulosic or non-cellulosic based paper and is capable of chromatographically or otherwise transporting a liquid. The absorbent material is selectively impregnated with a liquid mixture containing polymer or other material which penetrates the absorbent material such that this material becomes selectively impregnated cross-sectionally to a degree of up to 100% and such that the polymer or other impregnating material polymerises or solidifies, rendering the impregnated regions water impermeable. In Figure 10, regions 22 correspond to these water impermeable regions and together define the boundaries of the channel 4. Typically, the liquid material may be added using a printing technique, for example screen printing or ink jet printing, such that the regions to be impregnated may be pre-defined and the degree of impregnation across the surface of the absorbent material advantageously controlled to determine the shape and configuration of the impermeable regions, this defining the shape and configuration of the remaining permeable regions. Thus, channels 4 and sinks or reservoirs may be created and configured selectively within an assay module 2. Biosensor electrodes 16A and 16B, conductive tracks 18A and 18B and/or electrical connection interfaces 20 may be printed or otherwise deposited on the assay module 2 either prior to or after the selective impregnation process has been implemented, such that these conductive elements can perform their function to facilitate the performance of a test. The impregnated polymer serves to act as an electrical insulator, preventing electrical connection between the electrodes and/or conductive tracks.

An advantage of printing the conductive tracks and/or electrodes after formation of the channels by the selective impregnation of the doping polymer is that it eliminates the risk of the polymer printing process interfering with the already printed ink. As a further development of this approach, at the time of printing the polymer for the channels, a further polymer impregnated region could be defined to insulate the subsequently printed inks. Also, mediators or other material may be provided in the inks that could be specific to the particular assay being carried out. For example, the inks may be provided with reagents that promote, or are necessary for, the particular electrochemical/biochemical reactions that are to take place at the testing zone. In this scenario, printing the channels first would facilitate economies of scale in the manufacturing process: a stock of base devices with channels but no ink-formed electrodes and/or conductive tracks could be manufactured as a lot (they are all identical). Specific devices could then be finished by adding the appropriate electrode/conductive track inks for the particular electrochemical/biochemical reactions that are required (which may or may not be specific to the particular assay).

The assay module 2 may be coated with a water impermeable coating polymer 24, optionally transparent, as shown in Figure 10. The coating polymer may be bonded to the substrate, optionally by partial penetration of the coating polymer into the porous material such that the coating polymer penetrates from about 30% to about 70% of the thickness of the substrate, so as to define a continuous bibulous compartment using methods such as those described in US 6,573,108 B1.

The water impermeable polymer 24 may also be formed over the conductive tracks 18A and 18B to prevent spreading of the tracks, for example where they are formed by printing onto a porous substrate. In this case, the water impermeable polymer may be applied so as to penetrate by 100% of the thickness of the substrate in regions through which the conductive tracks 18A/18B pass, to completely encapsulate the conductive tracks. The polymer may be applied to one or both sides of the substrate in order to achieve this. The polymer may be a UV-curable polymer, for example.

There are two widely used types of immunochemical or other binding test format which may be used in the assay module 2.

The examples of test chemistry discussed above with reference to Figures 3 to 6 relate to the so-called "sandwich" assay, such as is used in laboratory based ELISA systems and which forms the basis of a number of commercial pregnancy tests. As described above, in a sandwich assay technique, two or more specific binders (7, 19) are used, one (19) immobilised or otherwise bound at the testing zone 6 and one (7) conjugated to the detectable label 11. In a positive test, target analyte molecules 9 cross-link the conjugated binder 5 to the immobilised binder 7 such that it may not be removed from the testing zone 6 in liquid moving downstream along the assay module 2.

An alternative assay format may be used, which is commonly referred to as the "competitive" assay. In a competitive assay, a controlled amount of a suitably modified analogue of the target analyte substance 9 is used to compete for a limited amount of specific binder (typically an antibody). When the concentration of analyte 9 is relatively low, analogue may bind in relatively high quantity to the specific binder. When the concentration of analyte 9 is relatively high, the analyte 9 may compete successfully with the analogue in binding to the specific binder and therefore specific binding of the analogue may be relatively in low.

Assay modules 2 of the present invention may incorporate either the sandwich or the competitive assay technique, or both. The embodiments described in detail in this specification are of the "sandwich" type.

The nature of many potential test and detection reagents that may be used is commonly such that they are thermally or otherwise unstable, especially when provided in liquid form. The test and detection reagents 10 and 12 used within an assay module 2 may be provided in dry form, typically by the initial application of reagents in liquid form followed by active drying of the reagents or by allowing them to dry naturally. Assay modules 2 manufactured in this way will afford thermal stability to the test and detection reagents 10 and 12, facilitating the use of the electrochemical test device 1 outside of the laboratory or other controlled environment by removing limitations in distribution, storage and handling that the need for refrigeration or cooling would impose.

Channels 4, 4A, 4B within the assay module 2 may be constructed in a number of ways. In a preferred embodiment of the present invention, the channels 4 are composed of a wettable absorbent matrix. This wettable matrix may be comprised of, for example but not by limitation, cellulose based paper, nitrocellulose membrane, woven or non-woven membranes or other sheet materials, non-cellulosic or synthetic papers. The absorbent material comprising the channels 4, 4A, 4B must be capable of transporting a liquid by chromatography, capillary action or other means, such that a liquid may flow from one portion of a channel 4, 4A, 4B towards another, for example from a deposition zone 8 to a waste channel 14. Channels 4, 4A, 4B within the assay module 2 may alternatively or additionally comprise hollow channels within a water impermeable material. Typically, this water impermeable material would be rigid and would be formed, for example but not by limitation, from a plastic or other polymer based material, for polystyrene or acrylic, or a ceramic material. Such materials are commonly used in the manufacture of microfluidic systems and plastics have been used previously to create hollow channels, as described, for example, in US Patent 6,551,841 and US Patent 6,767,510.

Using the same reference numerals as in Figure 2A, an embodiment of the invention for detecting pregnancy could incorporate an enzyme 11 conjugated to an antibody 7 that will bind specifically to the pregnancy marker 9, human chorionic gonadotrophin (hCG). The enzyme 11 could be peroxidase. This conjugate 5 could be deposited within the test reagent channel 4A at an appropriate location, for example added in liquid form and then dried. When a liquid urine sample is added to the assay module 2 at the deposition zone 8, it will be transported to and hydrate the dried conjugate 5, which will become mobile in the liquid phase within the test reagent channel 4A. The liquid phase will flow towards the testing zone 6. If there is a sufficient concentration of hCG in the sample, some of the hCG may be bound by the conjugate 5 as it flows towards the testing zone 6. The testing zone 6 contains a specific binder 19, typically a second antibody which is specific for the target substance 9 (hCG in this embodiment). This second specific binder 19 is immobilised or otherwise trapped at the testing zone 6. When the conjugate 5 reaches the test zone, some of the conjugate 5 which has bound to hCG may become bound at the test zone 6 by the second specific binder 19, which may also bind to hCG. Any conjugate 5 not bound at the test zone 6 is washed from the test zone 6 into the waste channel 14 by the continuing flow of liquid sample. Thus, if hCG is absent from the sample, or present at a sufficiently low concentration, then no conjugate 5, or very little conjugate 5, will become bound at the testing zone 6 and the conjugate 5 will move into the waste channel 14 with the liquid flow, away from the testing zone 6. Alternatively, if there is a sufficient concentration of hCG in the sample, then the antibody-peroxidase conjugate 5 will become and remain bound at the testing zone 6 such that it remains at the testing zone 6 after unbound conjugate 5 has been washed clear of the testing zone 6 and into the waste channel 14.

The electrodes 16A/16B for the assay module 2 can be formed by screen-printing or by ink-jet printing, for example. An example fabrication process is described below based on screen-printing of the electrodes onto a porous material comprising glass fibre paper (Whatman "Fusion 5"). The viability of this approach for the fabrication of paper biosensors was subsequently evaluated by depositing glucose oxidase on the working electrode and testing the resultant enzyme sensors, using a series of standard glucose solutions. For comparison, electrodes were also printed on cellulose paper.

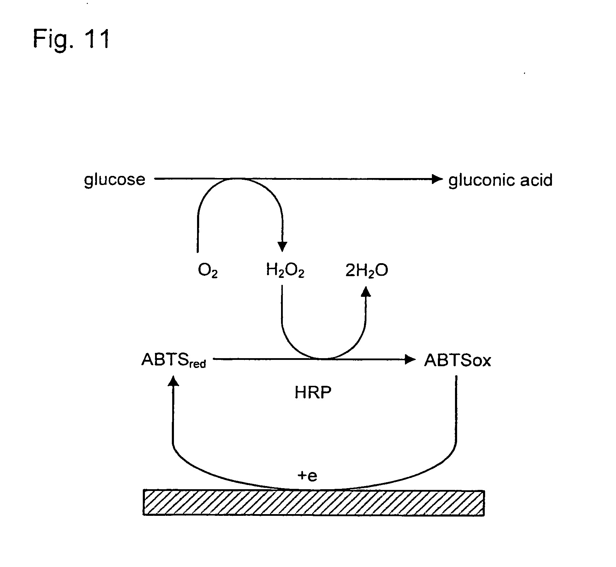

Before printing, a method for testing Fusion 5 and cellulose paper electrodes was devised. This was based on the electrochemical detection of oxidised 2,2'-azino-bis[3-ethylbenzthiazoline-6-sulfonic acid] (ABTS) generated in the reaction shown in Figure 11.

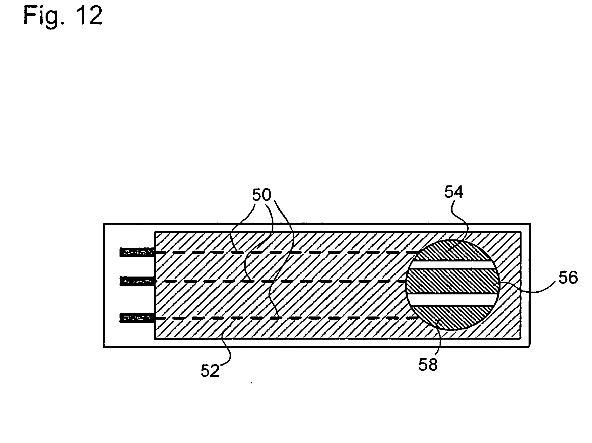

In essence, a 5mg/ml (1270U/ml) stock solution of glucose oxidase (GOx; Biozyme G03AC, Biozyme Laboratories, Blaenavon, Gwent, UK.) was made up in 0.1 M sodium phosphate buffer, pH 7.0. A 2 µl aliquot of the enzyme was applied directly to the working electrode 56 of 8 screen-printed Melinex electrodes, each comprising a central carbon working electrode 56, a carbon counter electrode 58 and a silver/silver chloride reference electrode 54 as shown schematically in Figure 12. Conductive tracks 50 were provided, leading to the reference, working and counter electrodes 54, 56, 58 over the porous material. The conductive tracks were electrically protected by an insulating shroud 52.

The remaining reaction components of the reaction were mixed as follows:

| | |
|---|---|
| 10 mM ABTS | 100 µl; |
| 222 U/ml HRP | 100 µl; |
| 205 mM sodium acetate buffer, pH 5.5 | 500 µl; |
| 1 M potassium chloride | 100 µl; |
| reverse osmosis water | 200 µl. |

Glucose solutions were made up at concentrations of 0, 2, 4, 8 and 12 mM in 205 mM sodium acetate buffer, pH5.5 containing 0.1 M potassium chloride.

The electrodes were connected to an AutoLab electrochemical analyser (Ecochemie, Utrecht, Netherlands). A 20 µl sample consisting of equal volumes of glucose and the reaction mix was pipetted over the electrode array and a potential of +150mV applied. The current over 300 seconds was recorded. The magnitude of the reducing current generated was detected to be proportional to the amount of glucose present, as expected.

The fabrication and testing of screen-printed paper electrodes is now described.

Electrodes were printed on Whatman cellulose paper, grade 1 chr (175 µm thickness) and Whatman Fusion 5 glass fibre paper in a two-stage process using a DEK 248 screen-printer. The Fusion 5 substrate had the following properties:
porosity of 1.65/100ml/in²;
thickness of 370 µm;
dry tensile strength of 25.2 N/15mm.

The Whatman cellulose paper was supplied by Cranfield Biotechnology Centre Institute of BioScience & TechnologyCBC.

The electrodes comprised three carbon conducting tracks 50 with a plain carbon working electrode 56, a carbon counter electrode 58 and a silver / silver chloride reference electrode 54. The conducting tracks 50, working electrode 56 and counter electrode 58 were printed as the first layer using Electrodag graphite ink 423SS (Acheson Colloids Co., Plymouth, Devon, UK). The reference electrode was printed in a second layer using Electrodag 10% silver chloride in silver ink 6038SS (Acheson Colloids Co.).

Both layers were printed using DEK stainless steel mesh screens with the following specifications:
325 wire count/inch;
45° mesh orientation;
solvent resistant emulsion, 13 µ thickness.

Close inspection of the electrodes showed that there was very little bleeding of the inks into the paper and that the definition of the carbon conducting tracks and all three component electrodes was good.

Initially, the electrodes were printed and tested using glucose oxidase and standard glucose solutions, as described above. However, it became apparent that the sample and reagents rapidly diffuse into the paper when applied to the electrode, resulting in a loss of signal. In order to circumvent this, while also providing both insulation and general protection, electrodes can be coated almost entirely with an insulating shroud 52, comprising for example a polymer or ink, leaving a small area for sample application.

As a first step, a UV curable polymer: Loctite 3106, was evaluated for its efficacy in preventing diffusion of liquid through both Fusion 5 and Whatman cellulose paper. The polymer was painted onto an area of each paper to form an open circle. The polymer was cured by exposure to UV light (UVAPRINT 100 CV) and Loctite 3106 subsequently applied to the rear side of the paper to cover the entire circle. After curing, a 20 µl volume of 3.3 mg/ml Brilliant Blue R-250 dye was applied to the centre of each circle and the degree of sample retention noted. Results indicated that in order to prevent diffusion of the dye into the paper, the Loctite had to be applied to both sides of the Fusion 5 or cellulose paper and the polymer cured for at least 5 minutes after each application.

Loctite polymer 3106 was then painted over the front and rear surfaces of the electrodes, leaving clear a 6 mm area over the electrode array for the sample application and the ends of the conducting tracks for connection to an electrochemical analyser. Each coat of polymer was cured by exposure to a UV light source for 5 minutes. It was necessary to apply a total of two coats of Loctite to each surface to prevent wicking of the test sample away from the site of application.

Initial tests using a multimeter indicated that the application of Loctite to the electrodes did not inhibit conductivity greatly.

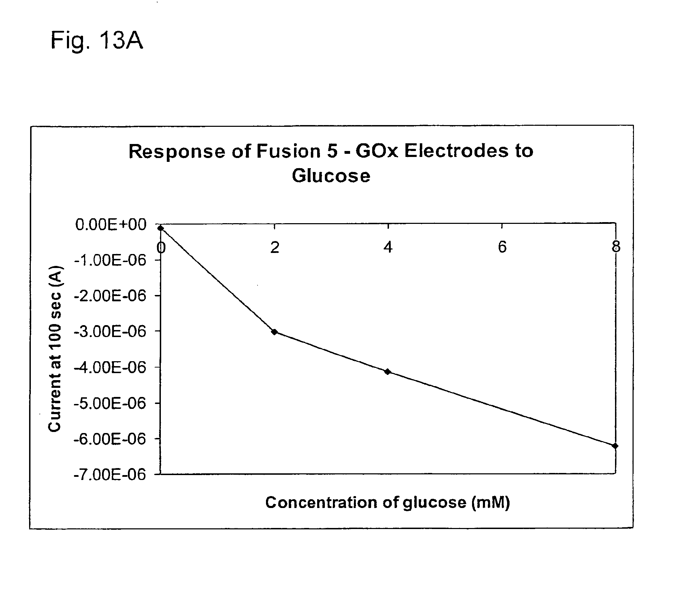

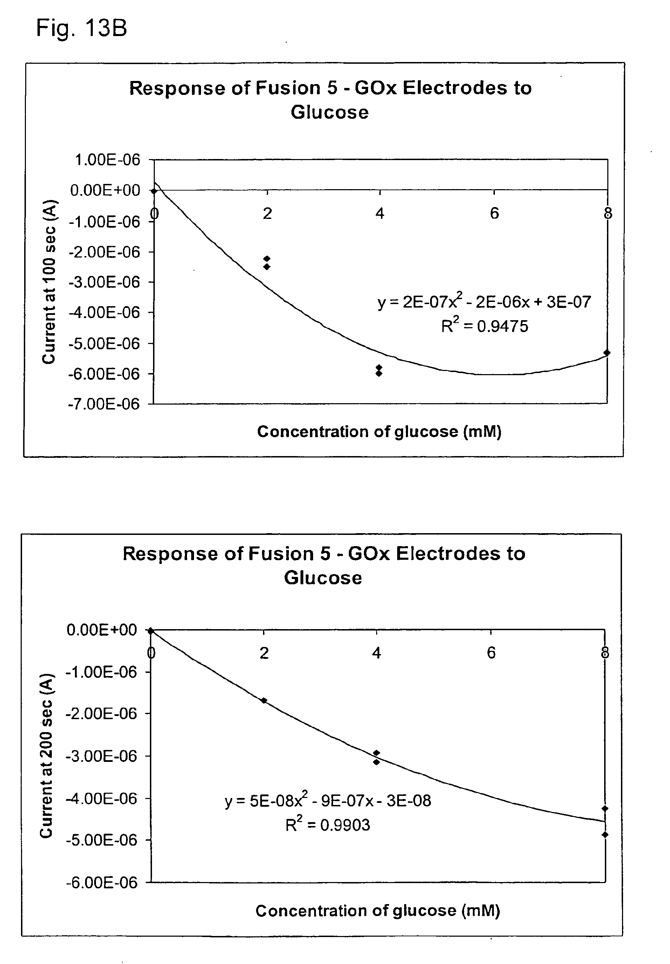

Glucose oxidase (GOx) was applied to the working electrode 56 of screen-printed Fusion 5 electrodes, coated with Loctite 3106 as described for the Melinex electrodes (above). Electrodes were subsequently tested with four concentrations of glucose 0, 2, 4 and 8 mM as described above and the current response at 100 and 200 seconds was plotted against glucose concentration. The results from two experiments performed on two different days are shown in Figures 13A and 13B (Figure 13A shows the results from the first experiment and Figure 13B shows the results from the second experiment).

Figures 13A and 13B show that the magnitude of the current response at both 100 and 200 seconds was directly related to the concentration of glucose in the sample. Plotting current at 200 seconds against glucose concentration appeared to generate the optimum calibration curve.

The above-described results demonstrate that screen-printing of electrodes on a cellulose or glass fibre paper substrate is a feasible technique for implementing the electrodes 16A/16B of the assay module 2 of the present invention. An acceptable print definition can be obtained for both of the above-mentioned paper types.

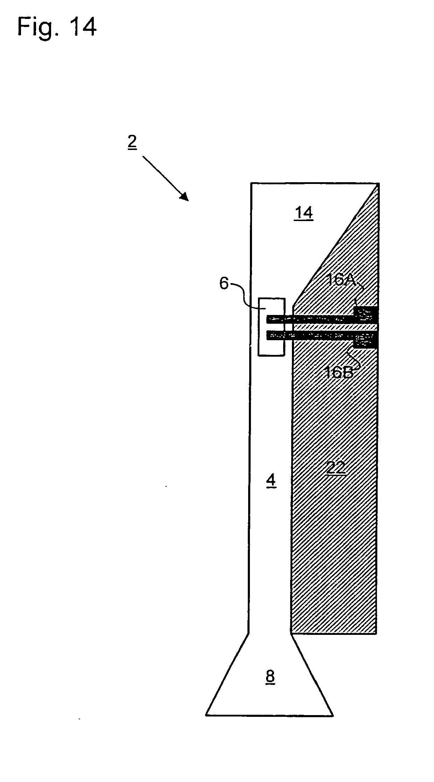

Figure 14 shows an alternative embodiment of the assay module 2. A channel 4 is formed in a porous substrate in a similar manner to the embodiment of Figure 10 discussed above. A water impermeable region 22 is created which defines the boundaries of the channel 4. In the present embodiment, both the deposition zone 8 and the waste channel/sink 14 are provided in a truncated, funnel-like form, which allows greater quantities of the capture antibody to be washed through the testing zone. Electrodes 16A/16B are provided to test the electrical properties of liquid in the testing zone 6, which, as discussed above, may be changed by the present of chemical compounds that are bound in the testing zone as a result of the presence of analyte in a sample added to the deposition zone 8.

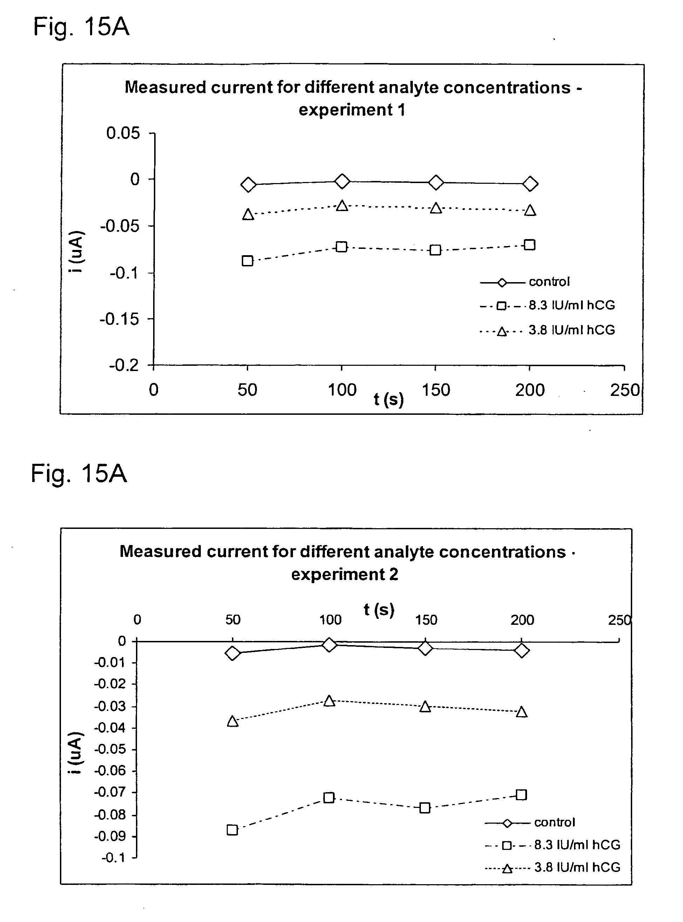

Figures 15A and 15B show the results of two separate experiments for determining the presence of hCG in a sample applied to the assay module 2 of Figure 14. In each case, the curves show the variation in electrical current i (measured by electrodes 16A/16B) as a function of time for different concentrations of hCG in the sample (zero for the control, 3.8 IU/ml and 8.3 IU/ml).

In these experiments, a capture antibody was biotinylated and added to the channel 4, along with HRP conjugate, at a position between the deposition zone 8 and the testing zone 6. A streptavidin solid phase that will bind to the biotinylated capture antibody is provided downstream at the testing zone 6. In use, a sample is added to the deposition zone 8 and the reagents (sample, biotinylated capture antibody and HRP conjugate) are washed down the channel 4. When the biotinylated capture antibody reaches the testing zone 6, it is bound by the streptavidin. If there is any analyte (hCG) present, it will cross-link HRP conjugate to the capture antibody, thus leading to HRP being immobilized in the testing zone 6. Unbound conjugate continues past the testing zone 6 to the waste channel/sink 14, away from the electrodes 16A/16B.

In further detail, in the immunoassay step, 10 µl of the biotinylated antibody (at a concentration of 30 µg/ml) and 10 µl of the HRP conjugate (at a concentration of 30 µg/ml) were added to the assay module 2 in a buffer solution of 0.1 % BSA (bovine serum albumine), 0.1 % SB 14 in pH 6.1 MES TRIS buffer (0.1 M) with a quantity of hCG in the buffer as indicated on the graphs (Figures 15A and 15B).

In the electrochemical measuring step, the following was added to the testing zone 6: an additional buffer of pH 4.1 citrate buffer with 1 mM ABTS and 0.2 mM H₂O₂. Electrical current was measured at a constant potential difference of 0.1 V for 200s, with open circuit prior to measurement.

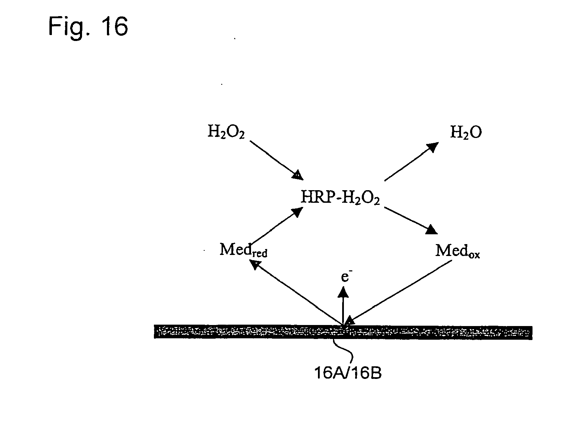

Figure 16 shows the reaction scheme in the testing zone 6 during the electrochemical measuring step. The electrode 16A/16B shown is a carbon screen printed electrode. Hydrogen peroxide forms a compound with HRP (HRP-H₂O₂). This compound oxidizes the ABTS mediator (Med_{red}) forming oxidized mediator (Medₒₓ) and H₂O. The oxidized mediator (Medₒₓ) is reduced at the electrode with an exchange of an electron (e⁻), which yields a measurable signal.

A second Ag/AgCl electrode (not shown in Figure 16) completes the electrical circuit and conserves total charge in the solution with the reaction:

AgCl (s) + e⁻ → Ag (s) + Cl⁻ (aq)

As can be seen from the curves in Figures 15A and 15B, when a control solution with no analyte is added to the assay module, almost no current is detected between the electrodes 16A and 16B. In contrast, where analyte is present at a concentration of 3.8 IU/ml, a relatively steady current of 0.04 µA is detected. Where analyte is present at a concentration of 8.3 IU/ml, a relatively steady current of about 0.08 µA (i.e. about twice what is measured for the lower analyte concentration) is detected. An electrical signal proportional to the concentration of analyte is thus measured.

## Claims

1. An assay module (2) for an electrochemical test device, said assay module (2) comprising:
at least one channel (4A,4B) for transporting a liquid to be tested from a deposition zone (8), where a sample of said liquid to be tested can be deposited, to a testing zone (6), the testing zone (6) being spaced apart from said deposition zone (8); and
electrodes (16A,16B) for measuring an electrical property of liquid in said testing zone (6), **characterized in that**:
said at least one channel (4A,4B) comprises a porous material capable of transporting said liquid chromatographically; and
the assay module further comprises a polymer that is impermeable to the liquid which the porous material is capable of transporting, incorporated into a part of the porous material so as to define a channel in the body of the porous material, the deposition zone and testing zone being provided on the porous material in the channel defined therein.

2. An assay module (2) according to claim 1, wherein at least one of said electrodes is formed by a printing technique onto said porous material, said printing technique being, optionally, screen printing or ink-jet printing; wherein:
said assay module (2) further comprises at least one conductive track between at least one of said electrodes (16A,16B) and a contact point on said assay module (2) away from said testing zone (6), so as to provide an electrical connection between said contact point and said testing zone (6); and
said assay module (2) further comprises a water-impermeable polymer impregnated into the porous material so as to at least partially encapsulate said at least one conductive track and/or said channel; or
said conductive track is formed over said polymer defining said channel.

3. An assay module (2) according to any one of the preceding claims, configured such that movement of said liquid to be tested from said deposition zone (8) to said testing zone (6) causes movement of one or more reagents to said testing zone (6), at least one of said reagents being such as to modify the electrical properties of liquid in said testing zone (6) if a target analyte is present in said liquid to be tested.

4. An assay module (2) according to claim 3, wherein a least one of said reagents is such as to become immobilized at said testing zone (6) if a target analyte is present in said liquid to be tested, said immobilization of said at least one of said reagents being such as to modify an electrical property of liquid in said testing zone (6).

5. An assay module (2) according to claim 3 or 4, wherein said one or more reagents is/are provided in said one or more channels (4A,4B) at a position or positions between said testing zone (6) and said deposition zone (8), prior to deposition of said liquid to be tested, wherein, optionally, at least one of said one or more reagents is/are so provided in dry form.

6. An assay module (2) according to claim 4 or 5, wherein said one or more channels (4A,4B) is/are configured to control the movement and/or action of said liquid to be tested and of said one or more reagents, and to deliver said liquid to be tested and said one or more reagents to said testing zone in an appropriate order and with appropriate timing to facilitate the performing of an electrochemical test in said testing zone (6) using said electrodes (16A,16B).

7. An assay module (2) according to any one of the preceding claims, wherein said electrodes (16A,16B) and/or conductive tracks leading to said electrodes (16A,16B) are formed by printing of a conductive ink and wherein said ink comprises at least one reagent which facilitates or is necessary for the electrochemical reaction due to take place at the testing zone (6).

8. An electrochemical test device (1), comprising:
an assay module (2) according to any one of the preceding claims and means for receiving an assay module (2) according to any one of the preceding claims; and
an analysis and display module (26) comprising:
a measurement section for interacting with said electrodes (16A,16B) in said assay module (2) in order to measure an electrical property of liquid in said testing zone (6);
an analysis section for obtaining information about the composition of said liquid to be tested by interpreting said electrical property of liquid in said testing zone (6) measured by said measurement section; and
a display section for displaying said information obtained by said analysis section.

9. An electrochemical test device (1) according to claim 8, wherein said display section is configured to display information obtained by said analysis section in textual and/or numerical form, wherein, optionally, said analysis section is capable of detecting at least three distinct concentrations or concentration ranges for a component in said liquid to be tested.

10. An electrochemical test device (1) according to claims 8 or 9, wherein said display section is configured to display at least three different images corresponding to each of the at least three distinct concentrations or concentration ranges detected by said analysis section.

11. An electrochemical test device (1) according to any one of claims 8 to 10, wherein said measurement section and electrodes (16A,16B) are configured to measure the current between the electrodes (16A,16B) at a constant potential difference.

12. An electrochemical test device (1) according to any one of claims 8 to 11, wherein said assay module (2) and said analysis and display module (26) each comprise an interface (32,34), said assay module (2) and said analysis and display module (26) being detachably connected to each other via said interface (32,34).

13. An electrochemical test device (1) according to any one of claims 8 to 12, further comprising:
a liquid reservoir for containing a liquid; and
a release mechanism for releasing said liquid into at least one of said at least one channel, wherein said liquid optionally comprises a reagent.

14. A method of testing a liquid sample to determine information about the composition of said liquid sample using an electrochemical test device (1) according to any one of claims 8 to 13, said method comprising:
depositing said liquid sample in said deposition zone (8); and
reading information displayed on said display section.

15. A method according to claim 14 when dependent on claim 12, further comprising:
between said depositing and reading steps, connecting said assay module (2) to said analysis and display module (26), via said interface (32,34).

## Patentansprüche

1. Testmodul (2) für eine elektrochemische Testvorrichtung, wobei das Testmodul (2) umfasst:
mindestens einen Kanal (4A, 4B) für den Transport einer zu testenden Flüssigkeit von einer Abscheidezone (8), wo eine Probe der zu testenden Flüssigkeit abgeschieden werden kann, zu einer Testzone (6), wobei die Testzone (6) von der Abscheidezone (8) beabstandet ist; und
Elektroden (16A, 16B) zum Messen einer elektrischen Eigenschaft von Flüssigkeit in der Testzone (6), **dadurch gekennzeichnet, dass**:
mindestens ein Kanal (4A, 4B) ein poröses Material umfasst, das die Flüssigkeit chromatographisch transportieren kann; und
das Testmodul weiterhin als Bestandteil eines Teils des porösen Materials ein für die Flüssigkeit, die das poröse Material transportieren kann, undurchlässiges Polymer umfasst, sodass ein Kanal im Körper des porösen Materials definiert wird, wobei die Abscheidezone und die Testzone auf dem porösen Material in dem darin ausgebildeten Kanal vorgesehen sind.

2. Testmodul (2) nach Anspruch 1, wobei mindestens eine der Elektroden mittels einer Drucktechnik auf dem porösen Material gebildet wird, wobei die Drucktechnik optional Siebdruck oder Tintenstrahldruck ist; wobei:
das Testmodul (2) ferner mindestens eine leitfähige Bahn zwischen mindestens einer der Elektroden (16A, 16B) und einem Kontaktpunkt auf dem Testmodul (2) umfasst, der von der Testzone (6) beabstandet ist, um eine elektrische Verbindung zwischen dem Kontaktpunkt und der Testzone (6) bereitzustellen; und
das Testmodul (2) ferner ein wasserundurchlässiges Polymer umfasst, das in das poröse Material eingebracht ist, zum zumindest teilweisen Einkapseln der mindestens einen leitfähigen Bahn und/oder des Kanals; oder
die leitende Bahn über dem den Kanal definierenden Polymer ausgebildet ist.

3. Testmodul (2) nach einem der vorhergehenden Ansprüche, welches derart konfiguriert ist, dass eine Bewegung der zu testenden Flüssigkeit aus der Abscheidezone (8) zu der Testzone (6) eine Bewegung von einem oder mehreren Reagenzien zu der Testzone (6) auslöst, wobei mindestens eines der Reagenzien die elektrischen Eigenschaften der Flüssigkeit in der Testzone (6) modifiziert, wenn ein Zielanalyt in der zu testenden Flüssigkeit vorhanden ist.

4. Testmodul (2) nach Anspruch 3, wobei mindestens eines der Reagenzien derart beschaffen ist, dass es an der Testzone (6) immobilisiert wird, wenn ein Zielanalyt in der zu testenden Flüssigkeit vorliegt, wobei die Immobilisierung des mindestens einen der Reagenzien derart ist, dass eine elektrische Eigenschaft der Flüssigkeit in der Testzone (6) modifiziert wird.

5. Testmodul (2) nach Anspruch 3 oder 4, wobei das eine oder die mehreren Reagenzien in dem einen oder den mehreren Kanälen (4A, 4B) an einer Position oder Positionen zwischen der Testzone (6) und der Abscheidezone (8) vor der Abscheidung der zu testenden Flüssigkeit bereitgestellt wird/werden, wobei optional mindestens eines des einen oder der mehreren Reagenzien derart in trockener Form bereitgestellt wird.

6. Testmodul (2) nach Anspruch 4 oder 5, wobei der eine oder die mehreren Kanäle (4A, 4B) konfiguriert ist/sind, die Bewegung und/oder Wirkung der zu testenden Flüssigkeit und des einen oder der mehreren Reagenzien zu steuern, und die zu testende Flüssigkeit und das eine oder die mehreren Reagenzien in einer geeigneten Reihenfolge und zu geeigneten Zeitpunkten zu der Testzone zu leiten, um die Durchführung eines elektrochemischen Tests in der Testzone (6) mit den Elektroden (16A, 16B) zu erleichtern.

7. Testmodul (2) nach einem der vorhergehenden Ansprüche, wobei die Elektroden (16A, 16B) und/oder leitenden Bahnen, die zu den Elektroden (16A, 16B) führen, durch Drucken einer leitfähigen Tinte gebildet sind, und wobei die Tinte mindestens ein Reagens enthält, das die elektrochemische Reaktion, die in der Testzone (6) stattfinden soll, erleichtert oder dafür erforderlich ist.

8. Elektrochemische Testvorrichtung (1), umfassend:
ein Testmodul (2) nach einem der vorhergehenden Ansprüche und Mittel zur Aufnahme eines Testmoduls (2) nach einem der vorhergehenden Ansprüche; und
ein Analyse- und Anzeigemodul (26) umfassend:
einen Messabschnitt für die Interaktion mit den Elektroden (16A, 16B) in dem Testmodul (2) zum Messen einer elektrischen Eigenschaft der Flüssigkeit in der Testzone (6);
einen Analyseabschnitt zum Erhalten von Informationen über die Zusammensetzung der zu testenden Flüssigkeit durch Interpretieren der von dem Messabschnitt gemessenen elektrischen Eigenschaft der Flüssigkeit in der Testzone (6); und
einen Anzeigeabschnitt zum Anzeigen der im Analyseabschnitt erhaltenen Informationen.

9. Elektrochemische Testvorrichtung (1) nach Anspruch 8, wobei der Anzeigeabschnitt konfiguriert ist, um die im Analyseabschnitt erhaltenen Informationen in Textform und/oder numerischer Form anzuzeigen, wobei optional der Analyseabschnitt mindestens drei unterschiedliche Konzentrationen bzw. Konzentrationsabschnitte einer Komponente in der zu testenden Flüssigkeit nachweisen kann.

10. Elektrochemische Testvorrichtung (1) nach einem der Ansprüche 8 oder 9, wobei der Anzeigeabschnitt konfiguriert ist, mindestens drei unterschiedliche Bilder, die jeweils einem der mindestens drei unterschiedlichen durch den Analyseabschnitt nachgewiesenen Konzentrationen bzw. Konzentrationsabschnitte entsprechen, anzuzeigen.

11. Elektrochemische Testvorrichtung (1) nach einem der Ansprüche 8 bis 10, wobei der Messabschnitt und die Elektroden (16A, 16B) konfiguriert sind, den Strom zwischen den Elektroden (16A, 16B) bei einer konstanten Potentialdifferenz zu messen.

12. Elektrochemische Testvorrichtung (1) nach einem der Ansprüche 8 bis 11, wobei das Testmodul (2) und das Analyse- und Anzeigemodul (26) jeweils eine Schnittstelle (32, 34) aufweisen, und das Testmodul (2) und das Analyse- und Anzeigemodul (26) über die Schnittstelle (32, 34) lösbar miteinander verbunden sind.

13. Elektrochemische Testvorrichtung (1) nach einem der Ansprüche 8 bis 12, ferner umfassend:
einen Flüssigkeitsbehälter zur Aufnahme einer Flüssigkeit; und
einen Freigabemechanismus zum Freisetzen der Flüssigkeit in mindestens einen der mindestens einen Kanäle, wobei die Flüssigkeit wahlweise ein Reagens umfasst.

14. Verfahren zum Testen einer flüssigen Probe zur Bestimmung von Informationen über die Zusammensetzung der flüssigen Probe unter Verwendung einer elektrochemischen Testvorrichtung (1) nach einem der Ansprüche 8 bis 13, wobei das Verfahren umfasst:
Abscheiden der flüssigen Probe in die Abscheidezone (8); und
Lesen von im Anzeigeabschnitt angezeigten Informationen.

15. Verfahren nach Anspruch 14 in Abhängigkeit von Anspruch 12, ferner umfassend:
Verbinden des Testmoduls (2) mit dem Analyse- und Anzeigemodul (26) über die Schnittstelle (32, 34) zwischen den Schritten des Abscheidens und des Lesens.

## Revendications

1. Module d'essai (2) pour un dispositif de test électrochimique, ledit module d'essai (2) comprenant :
au moins un canal (4A, 4B) pour transporter un liquide à tester, depuis une zone de dépôt (8), où un échantillon dudit liquide à tester peut être déposé, jusqu'à une zone de test (6), la zone de test (6) étant espacée de ladite zone de dépôt (8);
et
des électrodes (16A, 16B) pour mesurer une propriété électrique du liquide dans ladite zone de test (6), **caractérisé en ce que** :
ledit canal (4A, 4B), au nombre d'au moins un, est constitué d'un matériau poreux apte à transporter ledit liquide de façon chromatographique; et
le module d'essai comporte en outre un polymère qui est imperméable au liquide que le matériau poreux est apte à transporter, incorporé dans une partie du matériau poreux, de manière à définir un canal dans le corps du matériau poreux, la zone de dépôt et la zone de test étant prévues sur le matériau poreux dans le canal défini dans celui-ci.

2. Module d'essai (2) selon la revendication 1, dans lequel au moins l'une desdites électrodes est formée par une technique d'impression sur ledit matériau poreux, ladite technique d'impression étant, au choix, la sérigraphie ou l'impression par jet d'encre; où :
ledit module d'essai (2) comporte en outre au moins une piste conductrice entre au moins l'une desdites électrodes (16A, 16B) et un point de contact sur ledit module d'essai (2) situé à distance de ladite zone de test (6), de manière à établir une connexion électrique entre ledit point de contact et ladite zone de test (6); et
ledit module d'essai (2) comporte en outre un polymère imperméable à l'eau, imprégné dans le matériau poreux, de manière à encapsuler au moins partiellement ladite piste conductrice, au nombre d'au moins une, et/ou ledit canal; ou
ladite piste conductrice est formée sur ledit polymère en définissant ledit canal.

3. Module d'essai (2) selon l'une quelconque des revendications précédentes, configuré de telle façon que le mouvement dudit liquide à tester, de ladite zone de dépôt (8) à ladite zone de test (6), provoque le mouvement d'un ou plusieurs réactifs vers ladite zone de test (6), au moins un desdits réactifs étant tel qu'il modifie les propriétés électriques du liquide dans ladite zone de test (6), si l'analyte cible est présent dans ledit liquide à tester.

4. Module d'essai (2) selon la revendication 3, dans lequel au moins un desdits réactifs est tel qu'il est immobilisé à ladite zone de test (6) si un analyte cible est présent dans ledit liquide à tester, ladite immobilisation dudit réactif, au nombre d'au moins un, étant telle qu'elle modifie une propriété électrique du liquide dans ladite zone de test (6).

5. Module d'essai (2) selon la revendication 3 ou 4, dans lequel le ou les réactifs est/sont prévu(s) dans le ou les canaux (4A, 4B) à une position ou des positions entre ladite zone de test (6) et ladite zone de dépôt (8), avant le dépôt dudit liquide à tester, où, de manière facultative, au moins un réactif parmi le ou les réactifs est/sont prévu(s) sous une forme sèche.

6. Module d'essai (2) selon la revendication 4 ou 5, dans lequel le ou les canaux (4A, 4B) est/sont configuré(s) pour contrôler le mouvement et/ou l'action dudit liquide à tester et du ou des réactifs, et pour envoyer ledit liquide à tester et le ou les réactifs à ladite zone de test dans un ordre approprié et avec une temporisation appropriée pour faciliter la réalisation d'un test électrochimique dans ladite zone de test (6) en utilisant lesdites électrodes (16A, 16B).

7. Module d'essai (2) selon l'une quelconque des revendications précédentes, dans lequel lesdites électrodes (16A, 16B) et/ou les pistes conductrices menant auxdites électrodes (16A, 16B) sont formées en imprimant une encre conductrice, et dans lequel ladite encre comporte au moins un réactif qui facilite la réaction électrochimique devant avoir lieu dans la zone de test (6), ou est nécessaire à celle-ci.

8. Dispositif de test électrochimique (1), comprenant :
un module d'essai (2) selon l'une quelconque des revendications précédentes et un moyen pour recevoir un module d'essai (2) selon l'une quelconque des revendications précédentes; et
un module d'analyse et de visualisation (26), comprenant :
une section de mesure pour interagir avec lesdites électrodes (16A, 16B) dans ledit module d'essai (2) afin de mesurer une propriété électrique du liquide dans ladite zone de test (6);
une section d'analyse pour obtenir des informations sur la composition dudit liquide à tester, en interprétant ladite propriété électrique du liquide dans ladite zone de test (6), mesurée par ladite section de mesure; et
une section de visualisation pour visualiser lesdites informations obtenues par ladite section d'analyse.

9. Dispositif de test électrochimique (1) selon la revendication 8, dans lequel ladite section de visualisation est configurée pour visualiser les informations obtenues par ladite section d'analyse, sous forme de texte et/ou de chiffres, dans lequel, de manière facultative, ladite section d'analyse est apte à détecter au moins trois concentrations distinctes ou des plages de concentration pour un composant dans ledit liquide à tester.

10. Dispositif de test électrochimique (1) selon les revendications 8 ou 9, dans lequel ladite section de visualisation est configurée pour visualiser au moins trois images différentes correspondant à chacune des concentrations distinctes ou plages de concentration, au nombre d'au moins trois, détectées par ladite section d'analyse.

11. Dispositif de test électrochimique (1) selon l'une quelconque des revendications 8 à 10, dans lequel ladite section de mesure et les électrodes (16A, 16B) sont configurées pour mesurer le courant entre les électrodes (16A, 16B) à une différence de potentiel constante.

12. Dispositif de test électrochimique (1) selon l'une quelconque des revendications 8 à 11, dans lequel ledit module d'essai (2) et ledit module d'analyse et de visualisation (26) comportent chacun une interface (32, 34), ledit module d'essai (2) et ledit module d'analyse et de visualisation (26) pouvant être connectés de façon amovible l'un à l'autre, par l'intermédiaire de ladite interface (32, 34).

13. Dispositif de test électrochimique (1) selon l'une quelconque des revendications 8 à 12, comprenant en outre :
un réservoir à liquide destiné à contenir un liquide; et
un mécanisme de distribution pour distribuer ledit liquide dans au moins un desdits canaux, au nombre d'au moins un, dans lequel ledit liquide comporte de manière facultative un réactif.

14. Procédé de test d'un échantillon de liquide pour déterminer des informations sur la composition dudit échantillon de liquide, en utilisant un dispositif de test électrochimique (1) selon l'une quelconque des revendications 8 à 13, ledit procédé comprenant :
le dépôt dudit échantillon liquide dans ladite zone de dépôt (8); et
la lecture des informations visualisées sur ladite section de visualisation.

15. Procédé selon la revendication 14 lorsqu'elle dépend de la revendication 12, comprenant en outre :
entre lesdites étapes de dépôt et de lecture, la connexion dudit module d'essai (2) audit module d'analyse et de visualisation (26), par l'intermédiaire de ladite interface (32, 34).
